# EUROPEAN PATENT APPLICATION

(11) **EP 4 450 118 A1**
(43) Date of publication of application: **23.10.2024**
(21) Application number: 22916158.3
(22) Date of filing: 28.12.2022
(51) Int. Cl.: A61M 39/26, A61M 39/06, A61M 39/22

(54) **ACCESS PORT MEMBER AND PRODUCTION METHOD FOR SAME**

(30) Priority: 28.12.2021 JP 2021215333; 28.12.2021 JP 2021215334
(71) Applicant: Nikkiso Company Limited, Tokyo 150-6022 (JP)
(72) Inventor: ONO, Kazuhide, Makinohara-shi, Shizuoka 421-0496 (JP); KAWAJIRI, Hiroyuki, Makinohara-shi, Shizuoka 421-0496 (JP); OKAMOTO, Shingo, Makinohara-shi, Shizuoka 421-0496 (JP)
(74) Representative: von Hirschhausen, Helge
(86) International application number: PCT/JP2022/048459
(87) International publication number: WO 2023/127936

(57) **Abstract**

Provided is: an access port member capable of avoiding reduction in the durability of a valve caused by welding and fixing of a body to a cap part; and a method of manufacturing the access port member. The access port member includes: a body 8 internally including a flow path 8b for circulating blood and having an opening 8c communicating with the flow path 8b; a valve 9 made of an elastic member covering the opening 8c and attached to an attachment part 8d of the body 8, the valve 9 including a slit 9a that makes a transition from a closed state to an open state when pressed by a tip 11a of a syringe 11 capable of collecting blood or injecting a drug; a cap part 10 welded and fixed to the body 8 with the valve 9 interposed therebetween and the slit 9a facing outward; and a depression 8e formed at a position between a welding part Y1 and the attachment part 8d of the body 8.

## Description

### Technical Field

The present invention relates to an access port member and a method of manufacturing the access port member which is to be connected to a blood circuit for causing a patient's blood to extracorporeally circulate to enable collection of blood or injection of a drug with a connecting member such as a syringe.

### Background Art

In general, a blood circuit for causing a patient's blood to extracorporeally circulate used in a blood purification treatment such as a hemodialysis treatment is mainly comprised of a flexible tube, and an access port member (may also be referred as a "rubber button" or "coinfusion member") is connected to a predetermined portion of the blood circuit. For example, as disclosed in PTL 1, a known access port member may be mentioned, which includes: a body connected to a blood circuit; a valve in which a slit is formed; and a cap part fixed to the body with the valve interposed therebetween.

Because the known access port member is connected to a blood circuit, blood can be collected (blood collection) into a syringe or a liquid drug can be injected (drug injection) from a syringe by pressing the peripheral portion of the slit in the valve with the tip of a connecting member such as a syringe to cause a transition of the slit from a closed state to an open state by a pressing force. Since the valve is made of an elastic member such as a rubber material, the closed state of the slit can be maintained by the elastic force.

### Citation List

### Patent Literature

PTL 1: Japanese Unexamined Patent Application Publication (Translation of PCT Application) No. 2006-501976

### Summary of Invention

### Technical Problem

However, in a known access port member, a cap part is welded and fixed to a body by ultrasonic welding, thus molten resin produced at the time of welding may flow into an attachment part of a valve, and an excessive stress may be applied to the valve which reduces the durability thereof. At the time of welding, the air in the space surrounded by a welding part between the body and the cap part is compressed, thus an excessive compressive force is generated, and the durability of the valve may be reduced.

In the known access port member, the valve is made of an elastic member having a circular plan view, thus when the valve is attached to the body, the direction of the slit is not stable, and it is difficult to maintain a state of orienting in a previously set predetermined direction. Particularly, when a line segment slit is formed in the valve, and the line segment slit is desired to extend parallel to the direction (in other words, the extension direction of the flow path) in which blood flows, it is necessary to carefully attach the valve to the body while watching the direction of the slit, which causes a disadvantage of time consuming for attachment work.

The present invention has been conceived in view of the above circumstances and it is an object to provide an access port member and a method of manufacturing the access port member which are capable of avoiding reduction in the durability of the valve caused by welding and fixing of the cap part to the body, and an access port member which allows the valve to be easily attached to the attachment part of the body while extending the slit in a desired direction.

### Solution to Problem

An access port member according to an embodiment of the present invention includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part welded and fixed to the body with the valve interposed therebetween and the slit facing outward, the cap part having a locking part with a thread shape to be screwed and locked into a thread shape formed in the connecting member; and a depression formed at a position between a welding part of the body and the attachment part, or between a welding part of the cap part and a clamp region of the valve.

An access port member according to an embodiment of the present invention includes: a body having an opening; a valve made of an elastic member covering the opening and attached to an attachment part of the body in a press-fitted state, the valve including a line segment-shaped slit that makes a transition from a closed state to an open state when pressed by a tip of a connecting member capable of collecting blood or injecting a drug; and a cap part fixed to the body with the valve interposed therebetween and the slit facing outward, the cap part having a locking part with a thread shape to be screwed and locked into a thread shape formed in the connecting member. The valve is matched in shape and attached to the attachment part in the body, and has an asymmetric shape about an extension direction of a flow path and an orthogonal direction to the flow path.

### Advantageous Effects of Invention

According to the present invention, an access port member includes: a body having an opening; a valve made of an elastic member covering the opening and attached to an attachment part of the body, the valve including a slit that makes a transition from a closed state to an open state when pressed by a tip of a connecting member capable of collecting blood or injecting a drug; a cap part welded and fixed to the body with the valve interposed therebetween and the slit facing outward, the cap part having a locking part with a thread shape to be screwed and locked into a thread shape formed in the connecting member; and a depression formed at a position between a welding part of the body and the attachment part, or between a welding part of the cap part and a clamp region of the valve, thus it is possible to avoid reduction in the durability of the valve caused by welding and fixing of the cap part to the body.

According to the present invention, an access port member includes: a body having an opening; a valve made of an elastic member covering the opening and attached to an attachment part of the body in a press-fitted state, the valve including a line segment-shaped slit that makes a transition from a closed state to an open state when pressed by a tip of a connecting member capable of collecting blood or injecting a drug; and a cap part fixed to the body with the valve interposed therebetween and the slit facing outward, the cap part having a locking part with a thread shape to be screwed and locked into a thread shape formed in the connecting member. The valve is matched in shape and attached to the attachment part in the body, and has an asymmetric shape about the extension direction of the flow path and the orthogonal direction to the flow path, thus the valve can be easily attached to the attachment part of the body while extending the slit in a desired direction.

### Brief Description of Drawings

[Fig. 1] Fig. 1 schematically illustrates a blood circuit to which an access port member according to an embodiment of the present invention is applied.
[Fig. 2] Fig. 2 is a perspective view illustrating the access port member.
[Fig. 3] Fig. 3 shows a plan view and a side view illustrating the access port member.
[Fig. 4] Fig. 4 is a section taken along line IV-IV given in Fig. 3.
[Fig. 5] Fig. 5 is a section taken along line V-V given in Fig. 3.

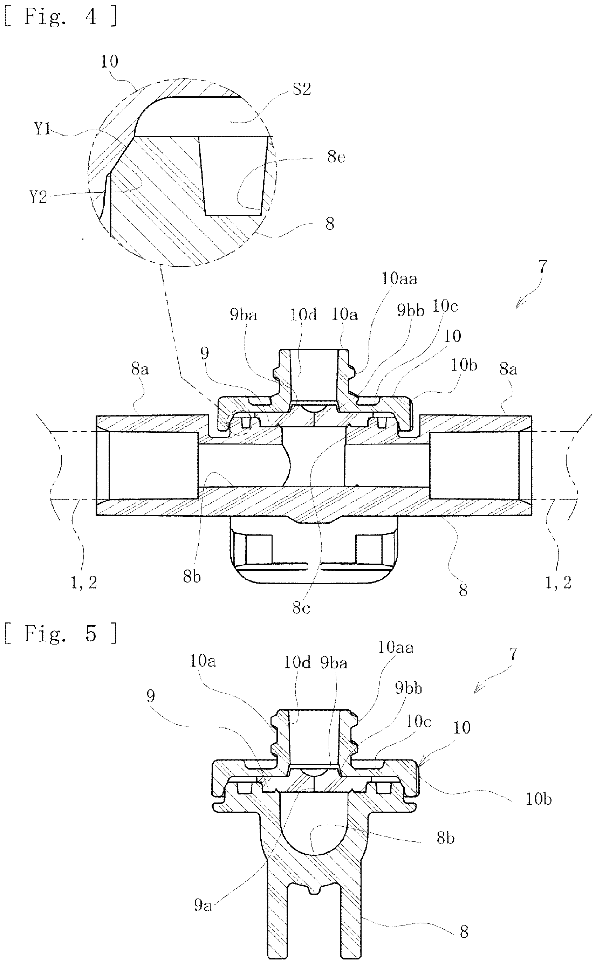

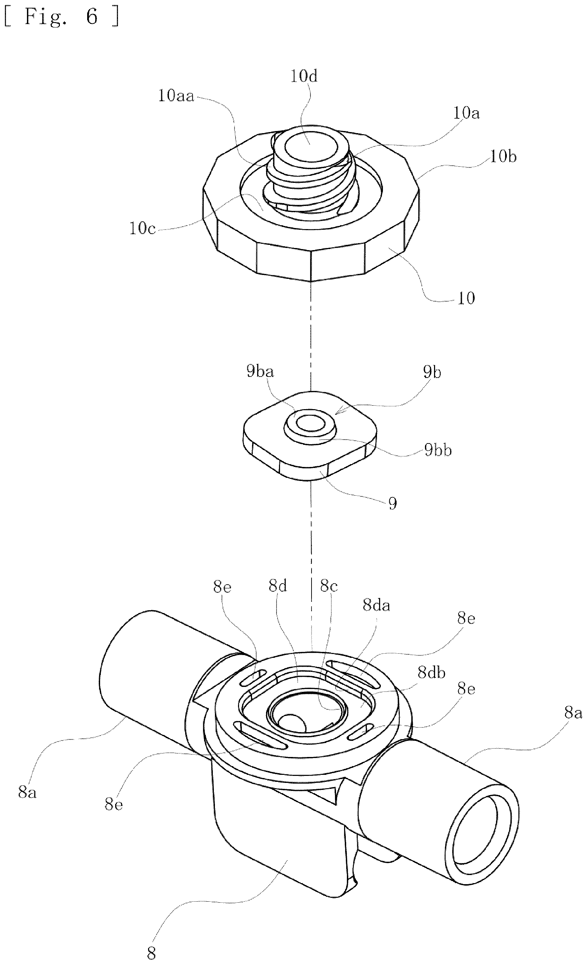

[Fig. 5] Fig. 6 is an exploded perspective view illustrating the access port member.

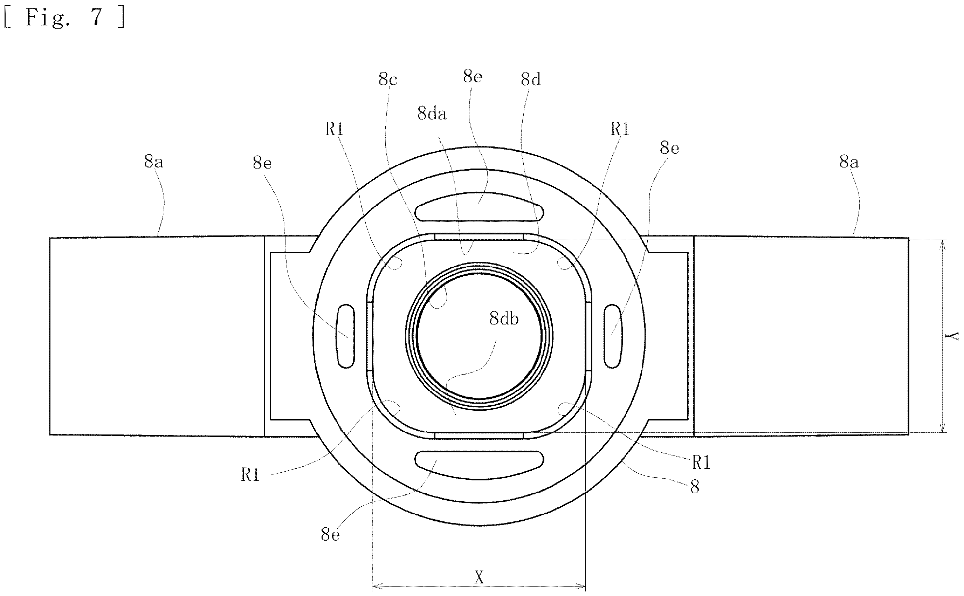

[Fig. 7] Fig. 7 is a plan view illustrating a body of the access port member.

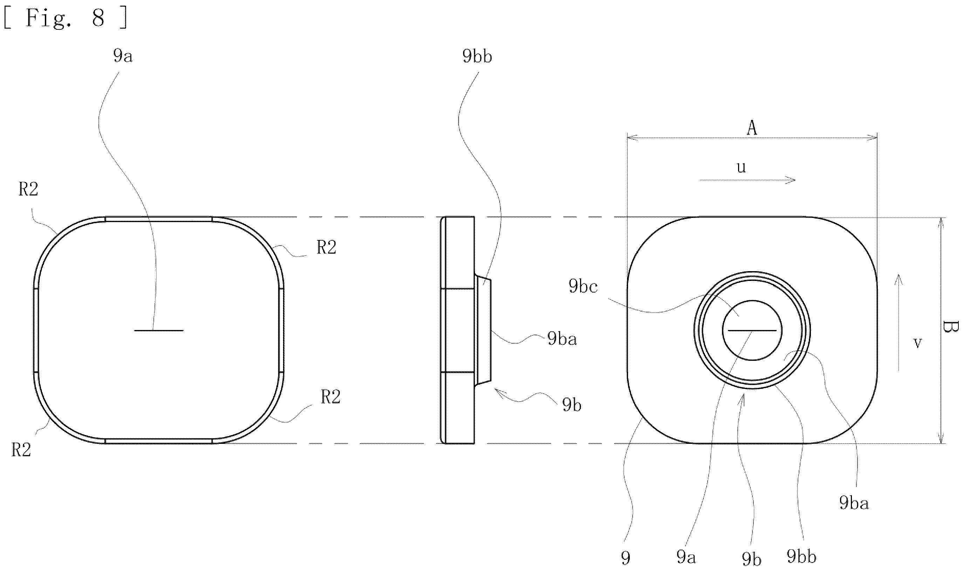

[Fig. 8] Fig. 8 is a three-view drawing illustrating a valve of the access port member.

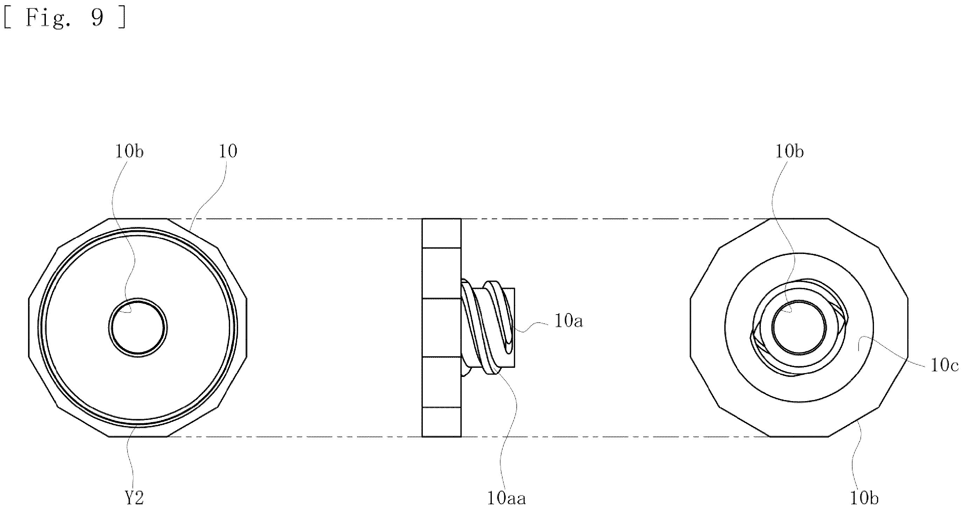

[Fig. 9] Fig. 9 is a three-view drawing illustrating a cap part of the access port member.

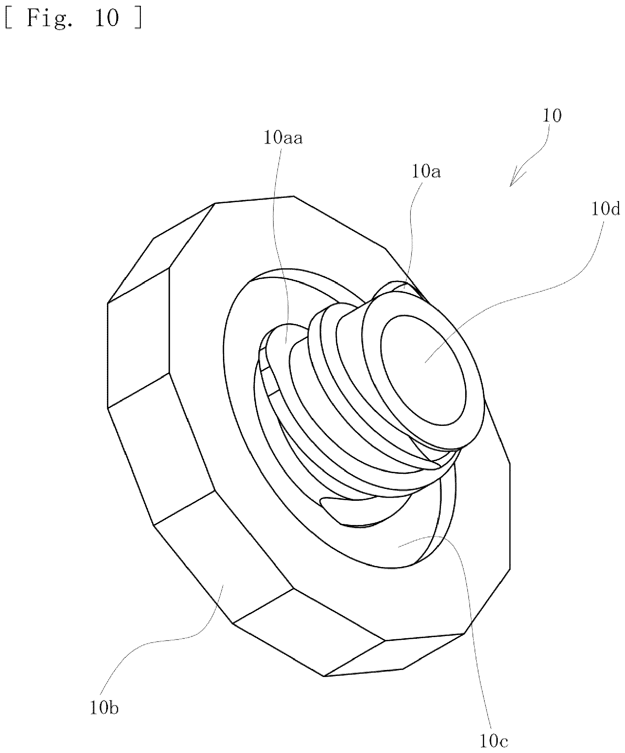

[Fig. 10] Fig. 10 is a perspective view of the cap seen from above.

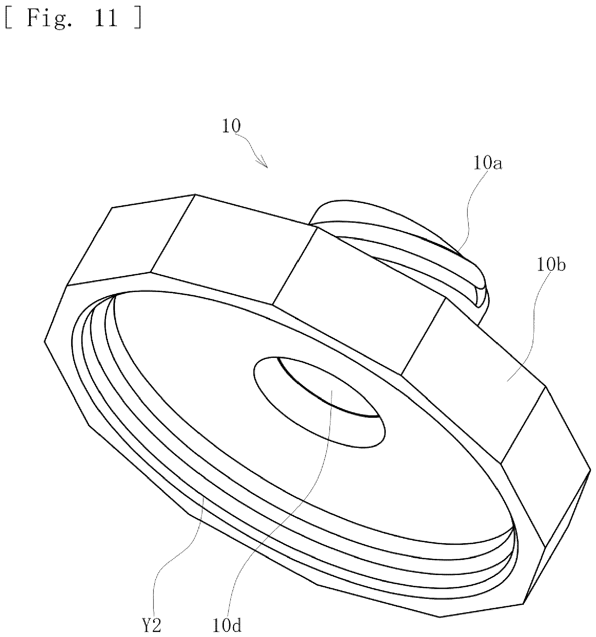

[Fig. 11] Fig. 11 is a perspective view of the cap seen from below.

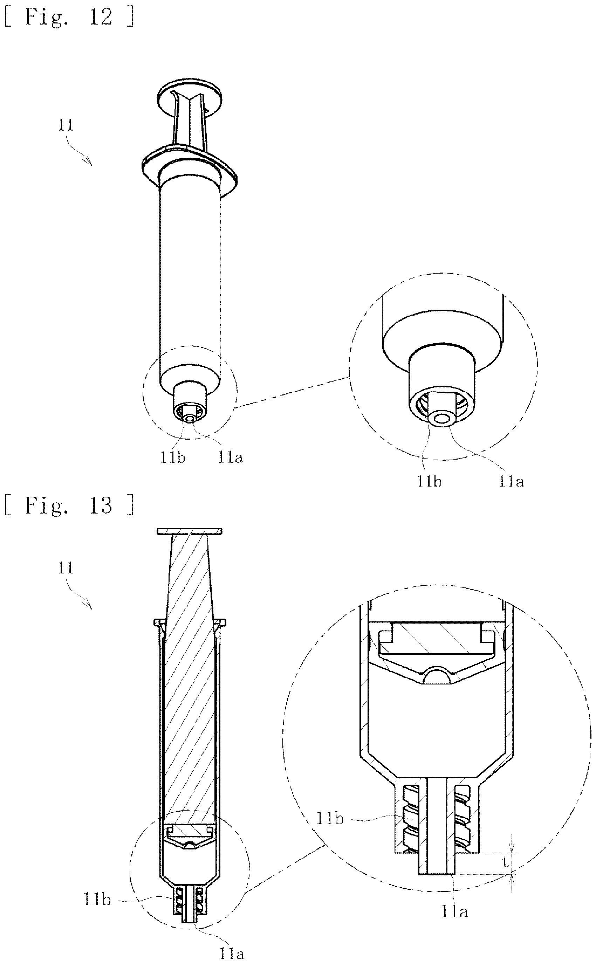

[Fig. 12] Fig. 12 is a perspective view illustrating a syringe (connecting member) applied to the access port member.
[Fig. 13] Fig. 13 is a vertical sectional view illustrating the syringe.

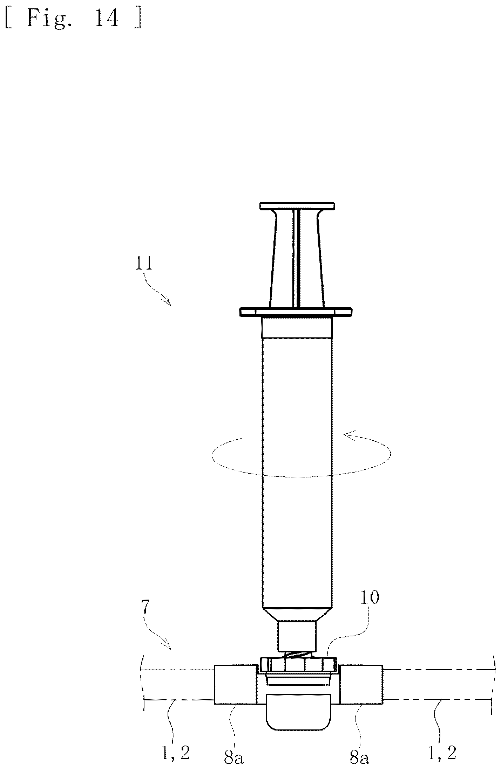

[Fig. 14] Fig. 14 is a schematic view illustrating a process of connecting the syringe to the access port.

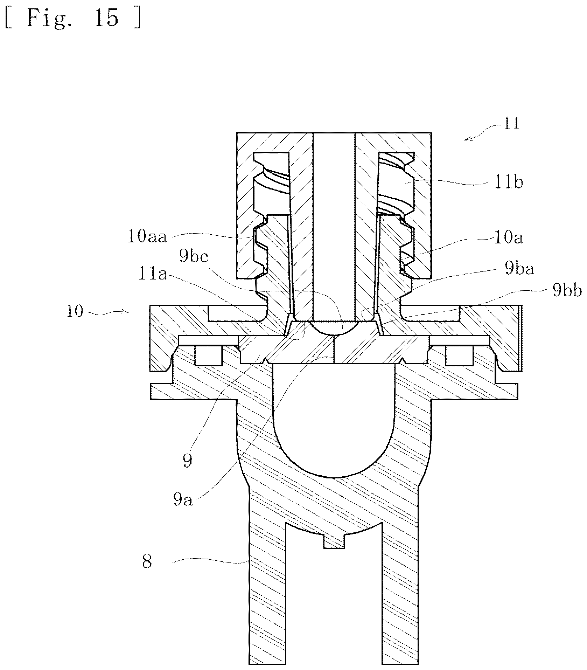

[Fig. 15] Fig. 15 is a sectional schematic view illustrating a state in which the tip of the syringe is brought into contact with the valve of the access port member.

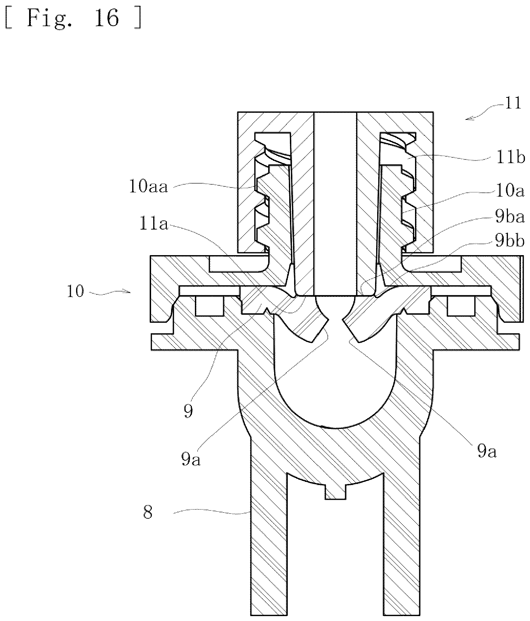

[Fig. 16] Fig. 16 is a sectional schematic view illustrating a state (a state in which the tip of the syringe is pressed against the tip surface of a rib in the valve) in which the tip of the syringe is pressed against the valve of the access port member.

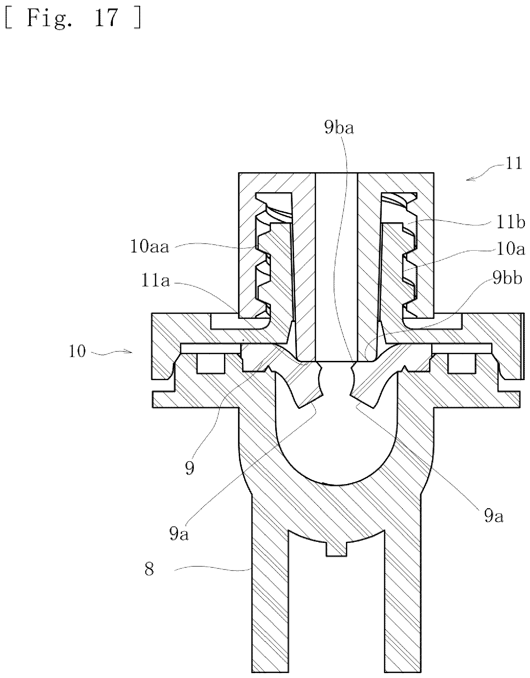

[Fig. 17] Fig. 17 is a sectional schematic view illustrating a state (a state in which the tip of the syringe is pressed against the tip surface and lateral surface of the rib in the valve) in which the tip of the syringe is pressed against the valve of the access port member.

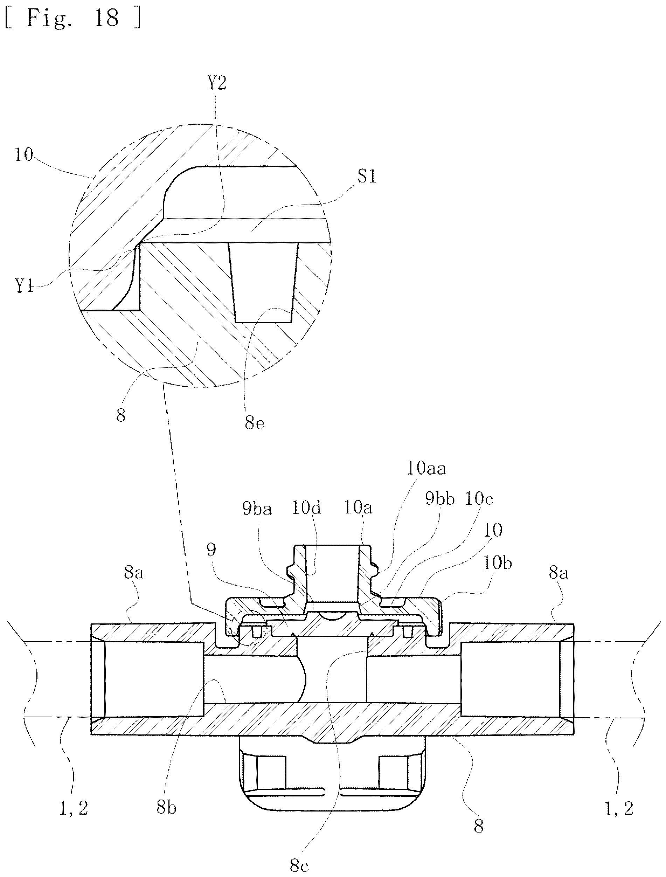

[Fig. 18] Fig. 18 is a sectional schematic view illustrating a state before the cap part of the access port member is welded and fixed.

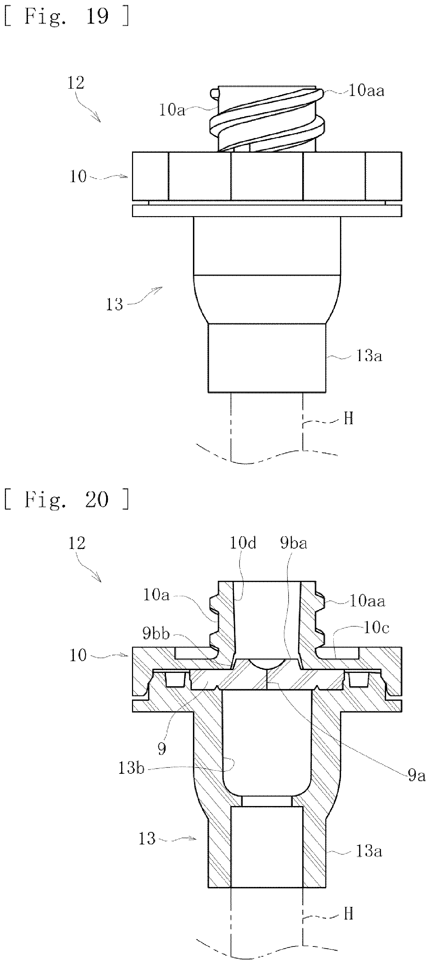

[Fig. 19] Fig. 19 is an external view illustrating an access port member according to another embodiment of the present invention.
[Fig. 20] Fig. 20 is a vertical sectional view illustrating the access port member according to another embodiment.

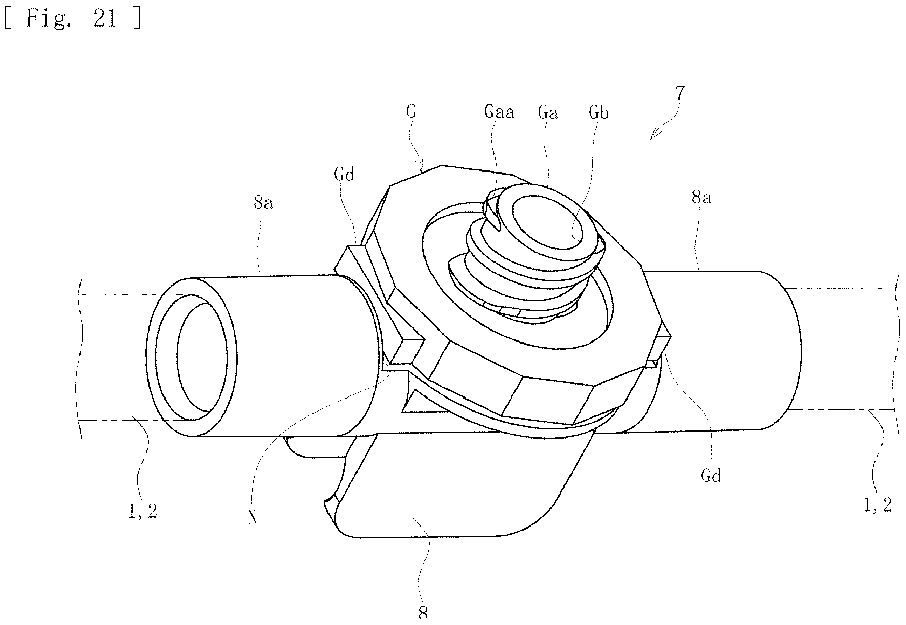

[Fig. 21] Fig. 21 is a perspective view illustrating an access port member according to still another embodiment of the present invention.

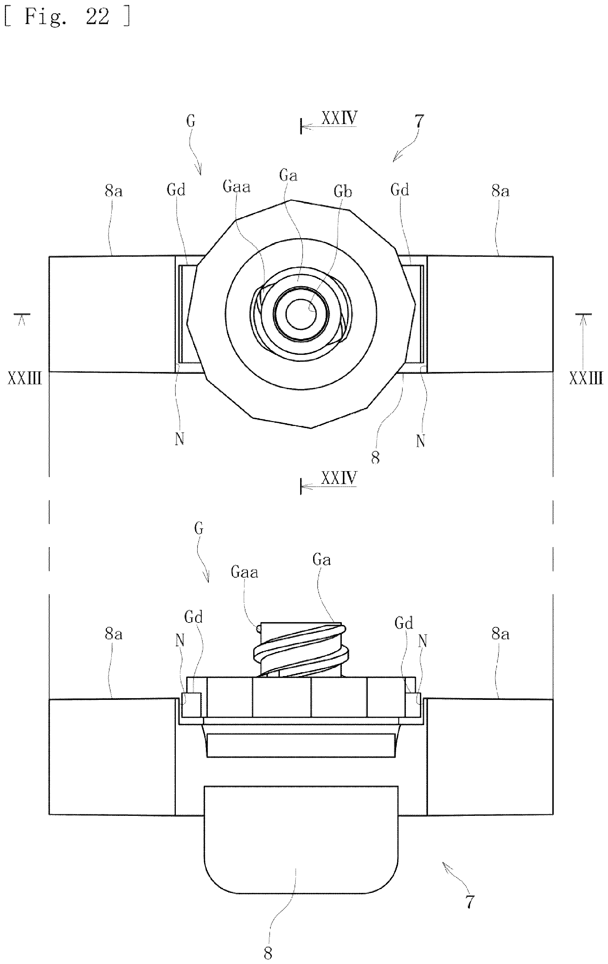

[Fig. 22] Fig. 22 shows a plan view and a front view illustrating the access port member.

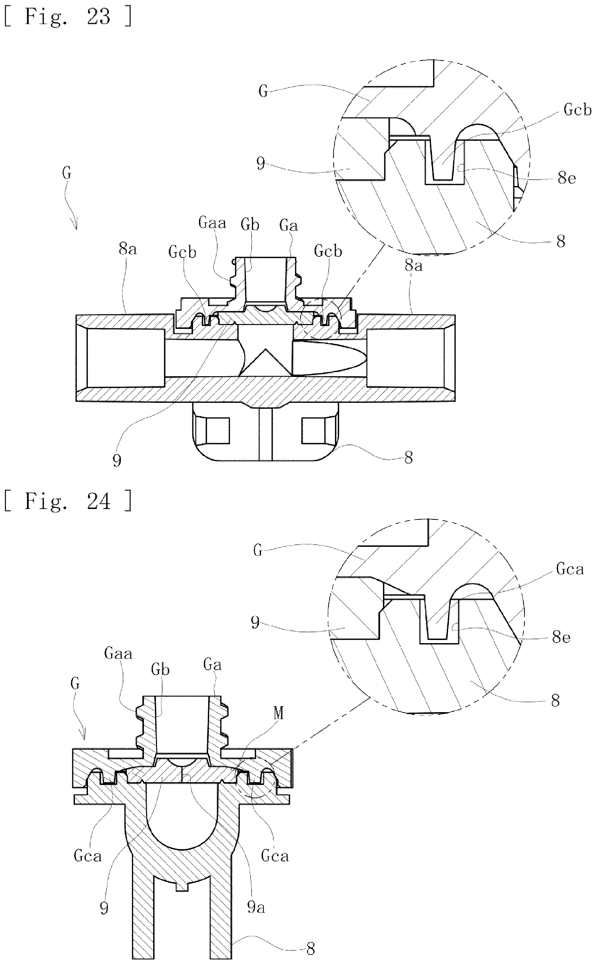

[Fig. 23] Fig. 23 is a section taken along line XXIII-XXIII given in Fig. 22.
[Fig. 24] Fig. 24 is a section taken along line XXIV-XXIV given in Fig. 22.

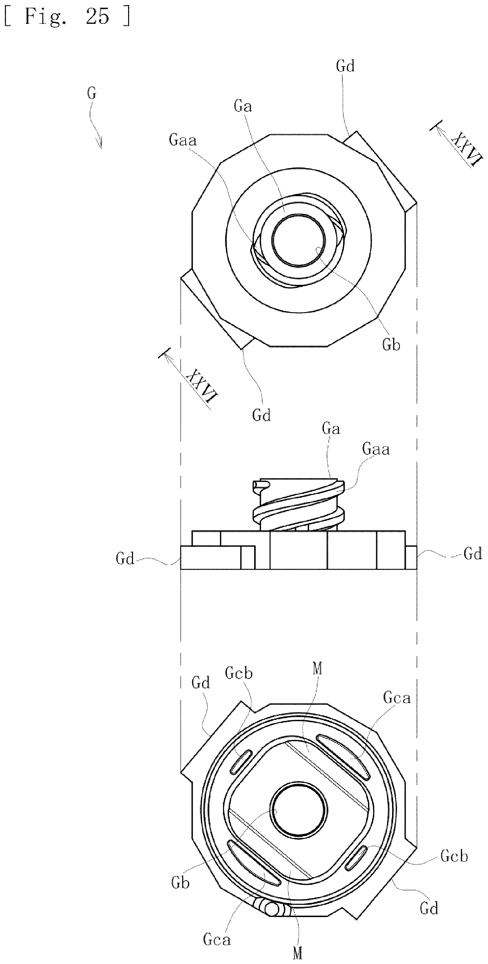

[Fig. 25] Fig. 25 is a three-view drawing illustrating a cap part in the access port member.

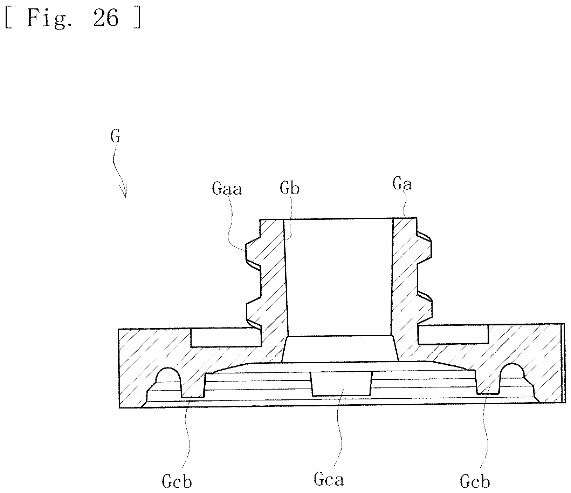

[Fig. 26] Fig. 26 is a section taken along line XXVI-XXVI given in Fig. 25.

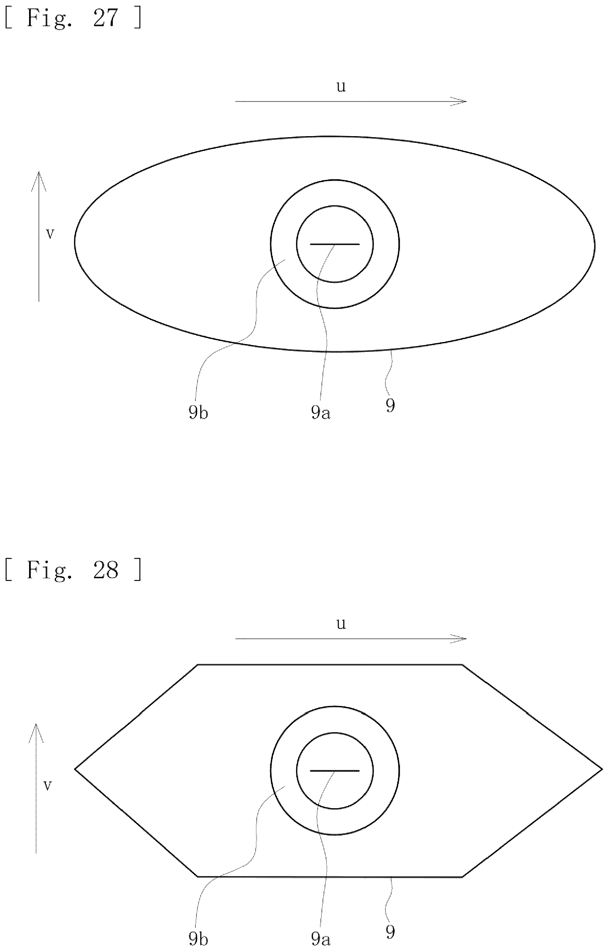

[Fig. 27] Fig. 27 is a plan view illustrating a valve of an access port member according to a second embodiment.
[Fig. 28] Fig. 28 is a plan view illustrating a valve of an access port member according to another embodiment.

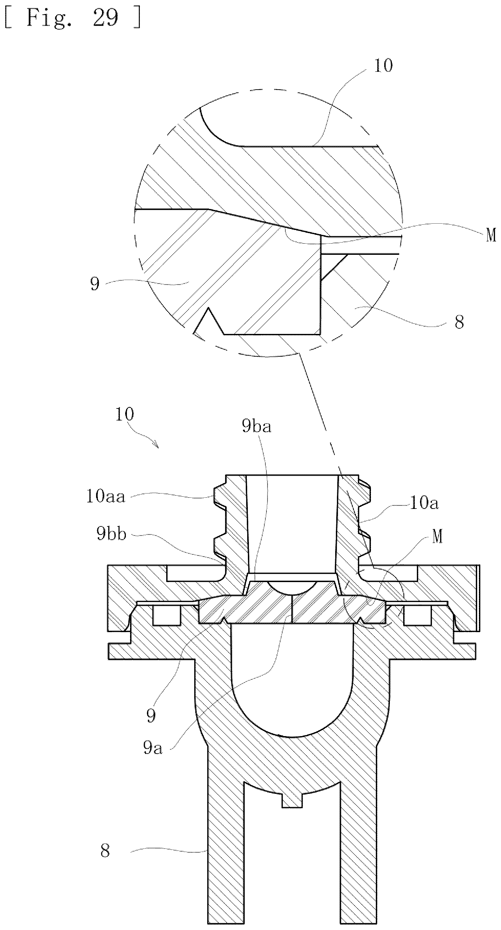

[Fig. 29] Fig. 29 is a section illustrating an access port member according to another embodiment.

### Description of Embodiments

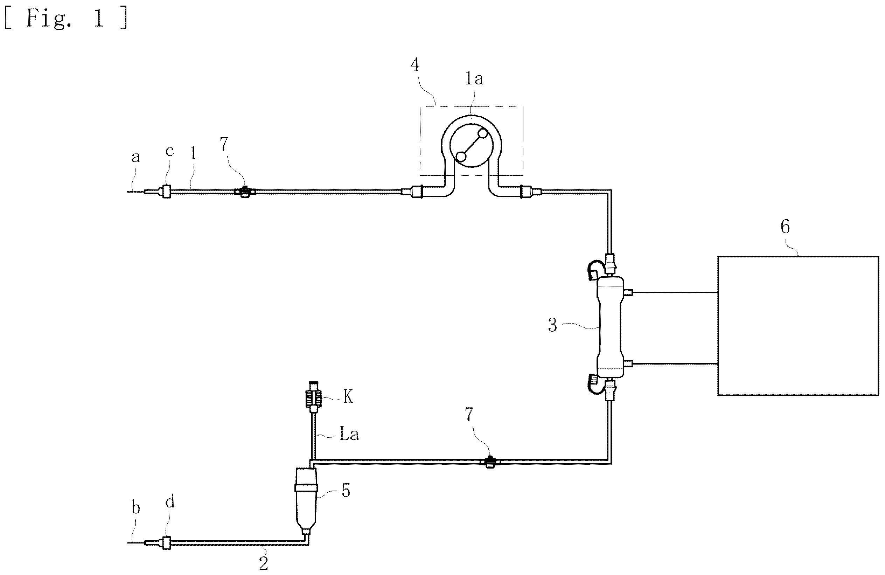

Hereinafter, an embodiment of the present invention will now be described specifically with reference to the drawings. An access port member according to a first embodiment is connected to a blood circuit for causing a patient's blood to extracorporeally circulate to enable a connecting member such as a syringe to collect blood or inject a drug, and as illustrated in Fig. 1, the blood circuit used in the present embodiment includes: a blood circuit having an arterial blood circuit 1 and a venous blood circuit 2; a dialyzer 3 as a blood purifier; a blood pump 4; and an air-trap chamber 5.

The arterial blood circuit 1 is formed of flexible tubes through which predetermined liquid is allowed to flow. The arterial blood circuit 1 is provided at the distal end thereof with a connector c, to which an arterial puncture needle a is attachable. The arterial blood circuit 1 is further provided with a squeezable tube 1a at a halfway position thereof. The squeezable tube 1a is attachable to the blood pump 4. The blood pump 4 is a peristaltic pump capable of delivering a priming solution or a patient's blood (liquid) in the blood circuit, and driving the blood pump 4 can cause a patient's blood to extracorporeally circulate.

The venous blood circuit 2 is formed of flexible tubes through which predetermined liquid is allowed to flow. The venous blood circuit 2 is provided at the distal end thereof with a connector d, to which a venous puncture needle b is attachable. The air-trap chamber 5 is connected to a halfway position of the venous blood circuit 2 to enable removal of the air contained in the blood that is under extracorporeal circulation in the blood circuit. A liquid drug infusion line La with a connecting part K attached to the distal end thereof for infusing liquid drug into the blood circuit extends from the top of the air-trap chamber 5. The blood circuit may not be provided with the liquid drug infusion line La at the top of the air-trap chamber 5.

The dialyzer 3 has, in a housing thereof, a plurality of hollow fibers each having microscopic holes (pores), and is capable of introducing dialysate from a dialysis device 6 as well as purifying the blood under extracorporeal circulation in the blood circuit by discharging drain liquid to the dialysis device 6. Specifically, the dialyzer 3 is connected between the arterial blood circuit 1 and the venous blood circuit 2, and the blood pump 4 is activated with the patient being punctured with the arterial puncture needle a and the venous puncture needle b, whereby the patient's blood is caused to extracorporeally circulate through the blood circuit (the arterial blood circuit 1 and the venous blood circuit 2) and the dialyzer 3 and is returned to the patient after being purified.

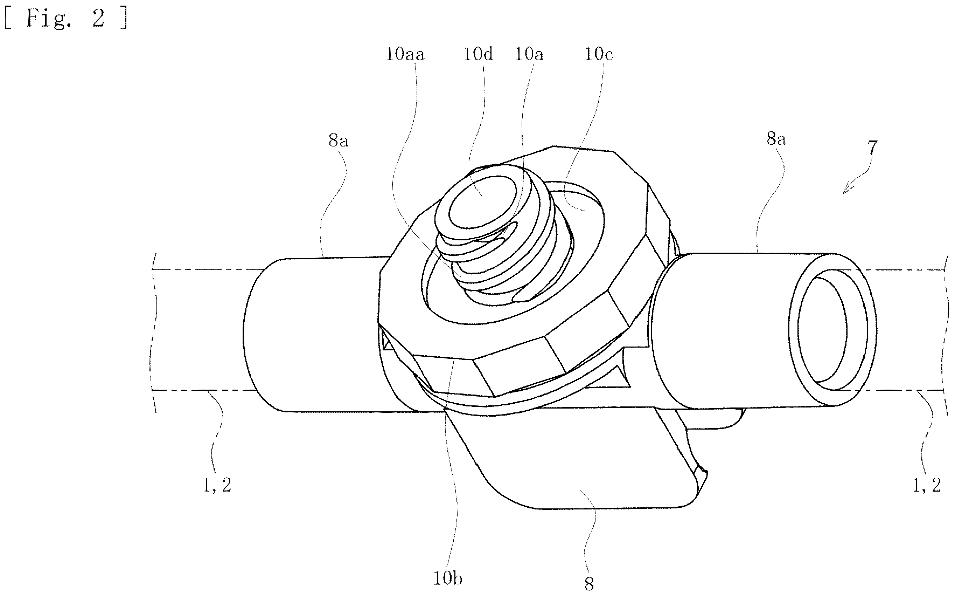

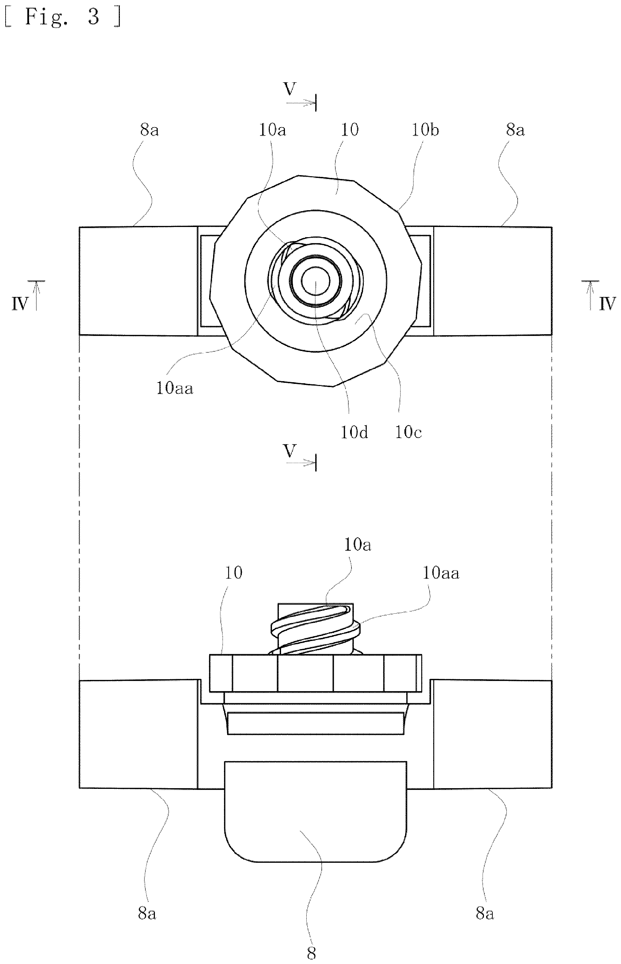

In the present embodiment, the access port member 7 is connected to a predetermined portion (for example, as illustrated, a portion between the connector c and the blood pump 4) of the arterial blood circuit 1, and a predetermined portion (for example, as illustrated, a portion between the dialyzer 3 and the air-trap chamber 5) of the venous blood circuit 2. The access port member 7 enables blood collection or drug injection for the blood that is under extracorporeal circulation in the blood circuit, and as illustrated in Figs. 2 to 6, includes a body 8, a valve 9, and a cap part 10. Note that the access port member 7 is not limited to the one connected to the blood circuit, and may be connected to, for example, the liquid drug infusion line La.

The body 8 is comprised of a resin molded part, and connected to a predetermined portion of the blood circuit (the arterial blood circuit 1 or the venous blood circuit 2) for causing a patient's blood to extracorporeally circulate, and as illustrated in Figs. 4 and 5, the body 8 internally includes a flow path 8b for circulating the blood, and an opening 8c communicating with the flow path 8b. As illustrated in Fig. 7, the body 8 includes: the connecting portions 8a formed at both ends thereof and connectable to respective flexible tubes constituting the blood circuit; the attachment part 8d formed at the opening edge of the opening 8c allowing the valve 9 to be attached; and the depressions 8e each serving as downgage formed around the attachment part 8d.

The attachment part 8d is in a depressed shape formed at the opening peripheral edge of the opening 8c, and has a shape copying the contour shape (shape copying a rectangle in the present embodiment) of the valve 9. The dimensions of the attachment part 8d are set to be slightly smaller than the external dimensions of the valve 9, allowing the valve 9 to be attached in a press-fitted state. Thus, the valve 9 is fixed with a compressive force applied inward from the outer peripheral edge, whereby the slit 9a is securely sealed.

The valve 9 is made of an elastic member (rubber material) covering the opening 8c and attached to the attachment part 8d of the body 8, and the slit 9a in a line segment shape is formed at a central portion. As illustrated in Fig. 8, the valve 9 according to the present embodiment has the contour of a rectangle having a long side A and a short side B in a plan view, and the slit 9a extends in the direction parallel to the long side A of the rectangle. The slit 9a makes a transition from a closed state (see Fig. 15) to an open state (see Figs. 16 and 17) when the front surface of the valve 9 is pressed by the tip 11a (see Fig. 13) of the syringe 11 (connecting member) capable of collecting blood or injecting a drug.

As illustrated in Figs. 12 to 14, the syringe 11 used in the present embodiment is capable of sucking liquid or discharging liquid, specifically, capable of collecting blood (blood collection) from the tip 11a by a suction operation, or capable of injecting a drug (drug injection) from the tip 11a by a discharge operation. The thread shape 11b screwable into the thread shape 10aa formed on the locking part 10a of the cap part 10 is integrally formed on the outer peripheral portion of the tip 11a of the syringe 11.

The valve 9 according to the present embodiment includes the annular rib 9b that projects along the periphery of the slit 9a. The rib 9b having the tip surface 9ba and the lateral surface 9bb is composed of a protrusion (thick wall portion) surrounding the periphery of the slit 9a in the front surface of the valve 9 and continuously projecting, and is formed copying the shape of the tip 11a of the syringe 11. As illustrated in Fig. 8, the rib 9b according to the present embodiment is circular in a plan view, and in the internal region 9bc thereof, the slit 9a in a line segment shape is formed. Thus, as illustrated in Figs. 15 to 17, the rib 9b is configured to, when the slit 9a makes a transition from a closed state to an open state, match the tip 11a of the syringe 11.

Specifically, as illustrated in Fig. 15, the internal region 9bc of the rib 9b is designed to be a thin wall portion, the slit 9a is formed in the thin wall portion, and the rib 9b is formed in the periphery of the thin wall portion, thereby increasing the intensity of the valve 9. Thus, since the slit 9a is formed in the thin wall portion of the internal region 9bc of the rib 9b, the slit 9a can make a transition from a closed state to an open state without applying a high pressing force to the rib 9b, and a sufficient intensity can be obtained by the rib 9b, thus even after repeated pressure to the rib 9b, a difference in level is prevented from occurring in the slit 9a.

Furthermore, as illustrated in Figs. 15 to 17, the rib 9b according to the present embodiment is configured to, when the slit 9a is in an open state, match and seal the tip 11a of the syringe 11 (connecting member), and in a process of insertion of the tip 11a of the syringe 11, during a transition of the slit 9a from a closed state (see Fig. 15) to an open state (see Figs. 16 and 17), and in a process of removal of the tip 11a of the syringe 11, during a transition of the slit 9a from an open state to a closed state, maintain a contact state with the tip 11a of the syringe 11.

Specifically, when the slit 9a is in a closed state, the tip 11a of the syringe 11 is brought into contact with the tip surface 9ba of the rib 9b (Fig. 15), and the syringe 11 is pushed in, thus the slit 9a makes a transition to an open state while the contact between the tip 11a of the syringe 11 and the tip surface 9ba of the rib 9b is being maintained (Fig. 16). When the syringe 11 is further pushed in, the tip 11a of the syringe 11 is brought into contact with the lateral surface 9bb subsequent to contact with the tip surface 9ba of the rib 9b (Fig. 17), thus sealing between the tip 11a of the syringe 11 and the rib 9b is maintained through the state illustrated in Figs. 15 to 17. However, even when a length t of projection (see Fig. 13) from the terminal of the thread shape 11b at the tip 11a of the syringe 11 is small, and the syringe 11 cannot be pushed in as shown in Fig. 17, the slit 9a can assume an open state while the contact between the tip 11a of the syringe 11 and the tip surface 9ba of the rib 9b being maintained as illustrated in Fig. 16. In replacement of the manner in which the tip 11a of the syringe 11 comes into contact with the tip surface of the rib 9b to maintain the sealing in this way, the tip 11a of the syringe 11 may come into contact with the lateral surface of the rib 9b to maintain the sealing.

Although the rib 9b according to the present embodiment is composed of the protrusion formed on the front surface of the valve 9, the rib 9b may be composed of the protrusion formed on the rear surface of the valve 9, or may be composed of the protrusion formed on both front and rear surfaces. Although the rib 9b according to the present embodiment is circular in a plan view, the rib 9b may be elliptical or rectangular in a plan view, and the slit 9a may be formed in the internal region 9bc. In addition to the manner in which the slit 9a is formed in a line segment shape, the slit 9a may be formed in a cross shape.

The cap part 10 is comprised of a component obtained by resin molding, and as illustrated in Figs. 4 and 5, is welded and fixed to the body 8 with the valve 9 interposed therebetween and the slit 9a facing outward. As illustrated in Figs. 9 to 11, the cap part 10 includes the locking part 10a, the outer peripheral edge 10b, the inner peripheral depression 10c, and the communication hole 10d. Specifically, the locking part 10a is formed to project along the central axis of the cap part 10, the inner peripheral depression 10c is formed around the locking part 10a, and the outer peripheral edge 10b having a large thickness is located on an outer diameter side of the inner peripheral depression 10c.

The locking part 10a internally includes the communication hole 10d through which the tip 11a of the syringe 11 is insertable and removable, and the spiral thread shape 10aa configured to match and screw into the thread shape 11b of the syringe 11 is integrally formed on the outer peripheral surface. Consequently, as illustrated in Fig. 14, the tip 11a of the syringe 11 is inserted into the communication hole 10d, and the syringe 11 is rotated, thus the thread shape 11b of the syringe 11 and the thread shape 10aa of the locking part 10a are screwed together and connected, whereby the syringe 11 can be locked (fixed) and connected to the access port member 7.

In particular, as illustrated in Fig. 4, in the access port member according to the present embodiment, a welding part Y1 of the body 8 and a welding part Y2 of the cap part 10 are matched (see Fig. 18), then the welding part Y1 and the welding part Y2 are caused to melt by applying ultrasonic waves thereto, thus the body 8 and the cap part 10 are welded and fixed (ultrasonic welded). Specifically, as illustrated in Figs. 9 and 11, the welding part Y2 of the cap part 10 is formed along the inner periphery of the outer peripheral edge 10b, and the welding part Y1 of the body 8 is formed in a circular shape to match the welding part Y2. As illustrated in Fig. 18, with the welding part Y1 and the welding part Y2 matched, performing ultrasonic welding causes the welding part Y1 and the welding part Y2 to be melted, and the body 8 and the cap part 10 are fixed.

In the body 8 according to the present embodiment, as illustrated in Figs. 6, 7, and 8, depressions 8e are formed at positions between the welding part Y1 and the attachment part 8d, the positions being on a plane located in the periphery of the attachment part 8d. As described above, such depression 8e serving as downgage formed in the body 8, and is formed at multiple locations corresponding to the sides (the long side A and the short side B) of the valve 9 attached to the attachment part 8d in the body 8. Although the depressions 8e according to the present embodiment are formed in the body 8, they may be formed between the welding part Y2 of the cap part 10 and the clamp region of the valve 9, or may be formed in both the body 8 and the cap part 10.

Specifically, the valve 9 according to the present embodiment is comprised of an elastic member formed (rectangular) in a rectangle having the long side A and the short side B in a plan view, the attachment part 8d of the valve 9 in the body 8 is formed (rectangular) in a rectangle in conformity with the shape of the valve 9, and the depressions 8e are formed corresponding to the outer positions of the sides (the long side A and the short side B) of the valve 9 attached to the attachment part 8d.

However, when the welding part Y1 of the body 8 and the welding part Y2 of the cap part 10 are matched before welding, as illustrated in Fig. 18, space S1 is formed above the depression 8e. When the cap part 10 is pressed against the body 8 in this state, while applying ultrasonic waves to the body 8 or the cap part 10, the welding part Y1 and the welding part Y2 are melted, and the volume of the closed space S1 is reduced and the air in the closed space S1 is compressed to create closed space S2 (see Fig. 4) with a small volume. Thus, as illustrated in Fig. 4, the cap part 10 is in a state of being fixed to the body 8.

Even if molten resin flows into the valve 9 from the welding part Y1 and the welding part Y2 at the time of welding, the molten resin is allowed to pass into the depressions 8e, thus the molten resin can be prevented from flowing to the valve 9. Consequently, it is possible to effectively avoid compression or deformation of the valve 9 due to the molten resin. In addition, at the time of welding, when the air in the closed space S1 is compressed, it is possible to avoid occurrence of an excessive compressive force because of the volume of the depressions 8e.

The access port member 7 according to the present embodiment includes: the body 8 having the opening 8c; the valve 9 made of an elastic member covering the opening 8c and attached to the attachment part 8d of the body 8, the valve 9 including the slit 9a that makes a transition from a closed state to an open state when pressed by the tip 11a of a connecting member such as the syringe 11 capable of collecting blood or injecting a drug; the cap part 10 welded and fixed to the body 8 with the valve 9 interposed therebetween and the slit 9a facing outward; and the depressions 8e formed at positions between the welding part Y1 of the body 8 and the attachment part 8d, or between the welding part Y2 of the cap part 10 and the clamp region of the valve 9, thus it is possible to avoid reduction in the durability of the valve 9 caused by welding and fixing of the cap part 10 to the body 8.

In particular, the body 8 is connected to a predetermined portion of the blood circuit for causing a patient's blood to extracorporeally circulate, thus the following effects can be achieved. Specifically, occurrence of a stagnation point on the rear surface of the valve 9 may cause blood clotting, thus such an occurrence needs to be reduced as much as possible. However, when the valve 9 is located closer to the flow route to prevent stagnation, the valve 9 cannot be sufficiently pressed by the tip 11a of the syringe 11, which makes it difficult to open the slit 9a. Thus, providing the valve 9 with the rib 9b as in the present embodiment allows the valve 9 to be sufficiently pressed by the tip 11a of the syringe 11, whereby both easiness of opening the slit 9a and less occurrence of a stagnation point can be achieved.

The valve 9 is made of an elastic member formed (rectangular) in a rectangular shape in a plan view, the attachment part 8d of the valve 9 in the body 8 is formed rectangular (rectangular shape) copying the shape of the valve 9, and the depression 8e is formed at multiple locations corresponding to the sides (the long side A and the short side B) of the valve 9 attached to the attachment part 8d in the body 8, thus the molten resin flowing from the welding parts Y1, Y2 can be reliably passed into the depression 8e. Note that the valve 9 may have another shape, and particularly when the valve 9 is circular in a plan view, the depression 8e may be a circular groove surrounding the outer periphery of the valve 9.

In addition, since the depression 8e serves as downgage of the body 8 or the cap part 10, both the effect (prevention of shrinkage) of the downgage, and the effect (maintenance of the durability of the valve 9) of passing the molten resin flowing from the welding parts Y1, Y2 into the depression 8e can be achieved. Specifically, when shrinkage occurs in part of the lateral surface or the bottom surface (see Figs. 6 and 7) in the attachment part 8d of the valve 9, the compressive force between the valve 9 and the attachment part 8d is reduced, and the closing force of the slit 9a may be reduced, thus such a problem can be prevented by the depression 8e that serves as downgage of the body 8 or the cap part 10. Furthermore, the welding part Y1 of the body 8 and the welding part Y2 of the cap part 10 are welded by being matched and melted, thus the cap part 10 can be fixed to the body 8 with high accuracy.

Also, the body 8 and the cap part 10 are welded by ultrasonic welding performed by applying ultrasonic waves to the welding parts (Y1, Y2), thus the body 8 and the cap part 10 can be fixed with ease and high accuracy. In the present embodiment, the cap part 10 is fixed to the body 8 by ultrasonic welding; however, the cap part 10 may be fixed to the body 8 by another welding method such as laser welding or heat welding.

In addition, the access port member 7 according to the present embodiment includes: the body 8 to be connected to a predetermined portion of a blood circuit for causing a patient's blood to extracorporeally circulate, the body 8 internally including the flow path 8b for circulating the blood and having the opening 8c communicating with the flow path 8b; the valve 9 made of an elastic member covering the opening 8c and attached to the attachment part 8d of the body 8, the valve 9 including the slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b integrally formed over the periphery of the slit 9a in the valve 9, thus the access port member 7 can facilitate connection work of the syringe 11 (connecting member) while preventing a failure in closing of the slit 9a.

In addition, the cap part 10 according to the present embodiment includes the locking part 10a with the thread shape 10aa to be screwed and locked into the thread shape 11b of the syringe 11 (connecting member), thus the following effects can be achieved. Specifically, since the dimensions of the locking part 10a are strictly defined by a standard, the slit 9a of the valve 9 may not be sufficiently pressed by the tip 11a of the syringe 11 (connecting member), thus the slit 9a may not be sufficiently opened. However, when the valve 9 is moved to the side where the syringe 11 is connected to prevent such a failure (for example, when the front surface of the valve 9 is superficialized to the side where the syringe 11 is connected), the valve 9 is made thicker, making it further difficult to open the slit 9a. When the valve 9 is made thinner and the valve 9 is superficialized, a liquid stagnation point occurs in greater number on the flow route side of the valve 9, and stagnation of blood and liquid drug is likely to occur, and in particular, when blood stagnates, blood clotting may occur. Thus, a structure needs to be created in which the slit 9a is reliably opened and stagnation is unlikely to occur. Thus, according to the present embodiment, providing the valve 9 with the rib 9b allows the tip 11a of the syringe 11 to sufficiently press the valve 9, whereby both easiness of opening the slit 9a and prevention of a stagnation point can be achieved.

The connecting member to be used is not limited to the syringe 11, and may be another connecting member (for example, a connecting member attached to the distal end of an infusion tube) which has a tip capable of pressing the rib 9b, and causes a transition of the slit 9a from a closed state to an open state by pressing to enable blood collection or drug injection. The valve 9 is not limited to the one having the contour of a rectangle in a plan view, and may be square, circular, elliptical or rectangular in a plan view.

In addition, the present invention is not limited to the one connected to a flow route for blood in the blood circuit, and may be connected to various flow routes (such as a liquid drug infusion line or a liquid drug delivery line serving as a flow route for a liquid drug) extending from the blood circuit. More specifically, in addition to the blood circuit for causing a patient's blood to extracorporeally circulate, the present invention is applicable to a circuit connected to a liquid delivery circuit, used for collection of blood flowing in the liquid delivery circuit, sampling of a liquid drug, and infusion of a liquid drug. For application to a liquid delivery circuit, an access port member may be used with one end thereof connected to an infusion bag and the other end connected to a connector coupled to an infusion line such as an indwelling needle or a blood circuit to enable drug injection and sampling, or an access port member may be used with connected to a liquid drug delivery circuit extended from a blood purifier such as the dialyzer 3 or various filters such as a blood adsorber, and a plasma separator to enable drug injection and sampling.

For application to a liquid delivery circuit, the access port member 12 having the configuration illustrated in Figs. 19 and 20 may be used. The access port member 12 includes: a body 13 having an opening 13b; a valve 9 made of an elastic member covering the opening 13b and attached to an attachment part of the body 13, the valve 9 including a slit 9a that makes a transition from a closed state to an open state when pressed by the tip 11a of the syringe 11 (connecting member) capable of collecting blood or injecting a drug; a cap part 10 fixed to the body 13 with the valve 9 interposed therebetween and the slit 9a facing outward, the cap part 10 having a locking part 10a with a thread shape 10aa to be screwed and locked into a thread shape 11b formed in the syringe 11; and a rib 9b integrally formed over the periphery of the slit 9a in the valve 9. The distal tip of a liquid delivery circuit H is to be connected to the connecting part 13a formed in the body 13. Note that the access port member 12 may be used instead of the connecting part K illustrated in Fig. 1.

As illustrated in Figs. 21 to 24, the access port member 7 may include a cap part G. The access port member 7 includes the same body 8 as illustrated in Figs. 6 to 8, and the valve 9 in which the slit 9a is formed, and the connecting portions 8a are to be connected to a flow route in a blood circuit or the like. The cap part G in this case includes a locking part Ga fixed to the body 8 with the valve 9 interposed therebetween and the slit 9a facing outward in an insertion hole Gb, the locking part Ga with a thread shape Gaa to be screwed and locked into a thread shape formed in a connecting member such as the syringe 11. The same inclined surface M as previously described is formed on the rear surface of the cap part G, and after the valve 9 is attached to the attachment part 8d, the cap part G is fixed to the body 8, causing the inclined surface M to press against the valve 9, thus the valve 9 is compressed in a direction in which the slit 9a is closed.

Furthermore, as illustrated in Figs. 25 and 26, in the cap part G, protrusions Gca, Gcb formed at positions corresponding to depressions 8e of the body 8, and projection parts Gd formed at positions corresponding to notches N (see Fig. 21) of the body 8 are integrally formed. As illustrated in Figs. 23 and 24, the protrusions Gca, Gcb have a projecting shape which is to be fitted into the depressions 8e when the cap part G is welded and fixed to the body 8, and protrusions Gca at positions adjacent to the long sides A of the valve 9, and protrusions Gcb at positions adjacent to the short sides B of the valve 9 are set to have different dimensions. The depressions 8e each serves as downgage, and include: depressions 8e corresponding to protrusions Gca adjacent to the long sides A; and depressions 8e corresponding to protrusions Gcb adjacent to the short sides B.

Thus, when the cap part G is attached to the attachment part 8d of the body 8, positioning in the rotational direction can be made by fitting the protrusions Gca, Gcb into the depressions 8e. However, when the positioning in the rotational direction is not correct at the time of attachment of the cap part G, a protrusion Gca having a larger size cannot be fitted into a depression 8e having a smaller size, and comes into contact with its peripheral surface, thus energy more than necessary is consumed at the time of ultrasonic welding, which makes it possible to inform of an error. In particular, the cap part G includes the inclined surface M that causes the valve 9 to be compressed in a closing direction of the slit 9a, thus positioning can be accurately made so that the inclined surface M becomes parallel to the slit 9a. Since the depressions 8e are formed in the body 8, the protrusions Gca, Gcb of the cap part G can be fitted thereinto, and positioning can be accurately made so that the inclined surface M becomes parallel to the slit 9a.

When the cap part G is welded and fixed to the body 8, if the positioning of the cap part G in the rotational direction is correct, each projection part Gd is opposed to the wall surface of a corresponding notch N as illustrated in Figs. 21 and 22. Thus, at the time of attachment of the cap part G, whether the positioning in the rotational direction is correct can be visually checked. As illustrated, the projection parts Gd are formed as a pair of right and left, but one of the pair may be formed, or alternatively, no projection part Gd may be formed.

Next, an access port member according to a second embodiment of the present invention will be described. An access port member according to the second embodiment is connected to a blood circuit for causing a patient's blood to extracorporeally circulate to enable a connecting member such as a syringe to collect blood or inject a drug, and as illustrated in Fig. 1, the blood circuit used in the present embodiment includes: a blood circuit having an arterial blood circuit 1 and a venous blood circuit 2; a dialyzer 3 as a blood purifier; a blood pump 4; and an air-trap chamber 5. The blood circuit to be used is the same as in the first embodiment, thus a detailed description is omitted, and a description is given using the drawings in common with those of the first embodiment.

In the present embodiment, the access port member 7 is connected to a predetermined portion (for example, as illustrated, a portion between the connector c and the blood pump 4) of the arterial blood circuit 1, and a predetermined portion (for example, as illustrated, a portion between the dialyzer 3 and the air-trap chamber 5) of the venous blood circuit 2. The access port member 7 enables blood collection or drug injection for the blood that is under extracorporeal circulation in the blood circuit, and as illustrated in Figs. 2 to 6, includes the body 8, the valve 9, and the cap part 10. Note that the access port member 7 is not limited to the one connected to the blood circuit, and may be connected to, for example, the liquid drug infusion line La.

The body 8 is comprised of a resin molded part, and connected to a predetermined portion of the blood circuit (the arterial blood circuit 1 or the venous blood circuit 2) for causing a patient's blood to extracorporeally circulate, and as illustrated in Figs. 4 and 5, the body 8 internally includes a flow path 8b for circulating the blood, and an opening 8c communicating with the flow path 8b. As illustrated in Fig. 7, the body 8 includes: the connecting portions 8a formed at both ends thereof and connectable to respective flexible tubes constituting the blood circuit; the attachment part 8d formed at the opening edge of the opening 8c allowing the valve 9 to be attached; and the depressions 8e each serving as downgage formed around the attachment part 8d.

The attachment part 8d is in a depressed shape formed at the opening peripheral edge of the opening 8c, and has a shape copying the contour shape (shape copying a rectangle in the present embodiment) of the valve 9. The dimensions of the attachment part 8d are set to be slightly smaller than the external dimensions of the valve 9, allowing the valve 9 to be attached in a press-fitted state. Thus, when the valve 9 is matched in shape and attached to the attachment part 8d in the body 8, the valve 9 is fixed with a compressive force applied inward from the outer peripheral edge, whereby the slit 9a is securely sealed.

The valve 9 is made of an elastic member (rubber material) covering the opening 8c and attached to the attachment part 8d of the body 8 in a press-fitted state, and the slit 9a in a line segment shape is formed at a central portion. As illustrated in Fig. 8, the valve 9 according to the present embodiment has the contour of a rectangle having a long side A and a short side B in a plan view, and the slit 9a extends in the direction parallel to the long side A of the rectangle. The slit 9a makes a transition from a closed state (see Fig. 15) to an open state (see Figs. 16 and 17) when the front surface of the valve 9 is pressed by a tip 11a (see Fig. 13) of a syringe 11 (connecting member) capable of collecting blood or injecting a drug.

In this manner, the valve 9 according to the present embodiment is a rectangle having a long side A and a short side B in a plan view as illustrated in Figs. 8 and 27, and has an asymmetric shape about the extension direction u of the flow path 8b and the orthogonal direction v to the flow path (rotation for 90° around the extension direction u and the orthogonal direction v gives variant shape), thus the slit 9a always extends in a predetermined direction by matching and attaching the valve 9 to the attachment part 8d. In particular, in the present embodiment, since the slit 9a extends in the direction parallel to the long side A of the rectangle which is the shape of the valve 9, the slit 9a extends in the extension direction of the flow path 8b by matching and attaching the valve 9 to the attachment part 8d.

When the valve 9 according to the present embodiment is matched and attached to the attachment part 8d in a press-fitted state, a compressive force is applied inward from the outer peripheral edge, and a compressive force in the direction in which the slit 9a is closed can be made larger than a compressive force in the opening direction because the long sides A have a greater pressure receiving area than the short sides B. In other words, when the valve 9 according to the present embodiment is matched and attached to the attachment part 8d in a press-fitted state, the slit 9a naturally extends in the extension direction of the flow path 8b, and a closed state of the slit 9a can be achieved with a stronger compressive force.

As illustrated in Figs. 12 to 14, the syringe 11 used in the present embodiment is capable of sucking liquid or discharging liquid, specifically, capable of collecting blood (blood collection) from the tip 11a by a suction operation, or capable of injecting a drug (drug injection) from the tip 11a by a discharge operation. The thread shape 11b screwable into the thread shape 10aa formed on the locking part 10a of the cap part 10 is integrally formed on the outer peripheral portion of the tip 11a of the syringe 11.

The valve 9 according to the present embodiment includes the annular rib 9b that projects along the periphery of the slit 9a. The rib 9b having the tip surface 9ba and the lateral surface 9bb is composed of a protrusion (thick wall portion) surrounding the periphery of the slit 9a in the front surface of the valve 9 and continuously projecting, and is formed copying the shape of the tip 11a of the syringe 11. As illustrated in Fig. 8, the rib 9b according to the present embodiment is circular in a plan view, and in the internal region 9bc thereof, the slit 9a in a line segment shape is formed. Thus, as illustrated in Figs. 15 to 17, the rib 9b is configured to, when the slit 9a makes a transition from a closed state to an open state, match the tip 11a of the syringe 11.

Specifically, as illustrated in Fig. 15, the internal region 9bc of the rib 9b is designed to be a thin wall portion, the slit 9a is formed in the thin wall portion, and the rib 9b is formed in the periphery of the thin wall portion, thereby increasing the intensity of the valve 9. Thus, since the slit 9a is formed in the thin wall portion of the internal region 9bc of the rib 9b, the slit 9a can make a transition from a closed state to an open state without applying a high pressing force to the rib 9b, and a sufficient intensity can be obtained by the rib 9b, thus even after repeated pressure to the rib 9b, a difference in level is prevented from occurring in the slit 9a.

Furthermore, as illustrated in Figs. 15 to 17, the rib 9b according to the present embodiment is configured to, when the slit 9a is in an open state, match and seal the tip 11a of the syringe 11 (connecting member), and in a process of insertion of the tip 11a of the syringe 11, during a transition of the slit 9a from a closed state (see Fig. 15) to an open state (see Figs. 16 and 17), and in a process of removal of the tip 11a of the syringe 11, during a transition of the slit 9a from an open state to a closed state, maintain a contact state with the tip 11a of the syringe 11.

Specifically, when the slit 9a is in a closed state, the tip 11a of the syringe 11 is brought into contact with the tip surface 9ba of the rib 9b (Fig. 15), and the syringe 11 is pushed in, thus the slit 9a makes a transition to an open state while the contact between the tip 11a of the syringe 11 and the tip surface 9ba of the rib 9b is being maintained (Fig. 16). When the syringe 11 is further pushed in, the tip 11a of the syringe 11 is brought into contact with the lateral surface 9bb subsequent to contact with the tip surface 9ba of the rib 9b (Fig. 17), thus sealing between the tip 11a of the syringe 11 and the rib 9b is maintained through the state illustrated in Figs. 15 to 17. However, even when a length t of projection (see Fig. 13) from the terminal of the thread shape 11b at the tip 11a of the syringe 11 is small, and the syringe 11 cannot be pushed in as illustrated in Fig. 17, the slit 9a can assume an open state while the contact between the tip 11a of the syringe 11 and the tip surface 9ba of the rib 9b being maintained as illustrated in Fig. 16. In replacement of the manner in which the tip 11a of the syringe 11 comes into contact with the tip surface of the rib 9b to maintain the sealing in this way, the tip 11a of the syringe 11 may come into contact with the lateral surface of the rib 9b to maintain the sealing.

Although the rib 9b according to the present embodiment is composed of the protrusion formed on the front surface of the valve 9, the rib 9b may be composed of the protrusion formed on the rear surface of the valve 9, or may be composed of the protrusion formed on both front and rear surfaces. Although the rib 9b according to the present embodiment is circular in a plan view, the rib 9b may be elliptical or rectangular in a plan view, and the slit 9a may be formed in the internal region 9bc.

The cap part 10 is comprised of a component obtained by resin molding, and as illustrated in Figs. 4 and 5, is welded and fixed to the body 8 with the valve 9 interposed therebetween and the slit 9a facing outward. As illustrated in Figs. 9 to 11, the cap part 10 includes a locking part 10a, an outer peripheral edge 10b, an inner peripheral depression 10c, and a communication hole 10d. Specifically, the locking part 10a is formed to project along the central axis of the cap part 10, the inner peripheral depression 10c is formed around the locking part 10a, and the outer peripheral edge 10b having a large thickness is located on an outer diameter side of the inner peripheral depression 10c.

The locking part 10a internally includes the communication hole 10d through which the tip 11a of the syringe 11 is insertable and removable, and a spiral thread shape 10aa configured to match and screw into the thread shape 11b of the syringe 11 is integrally formed on the outer peripheral surface. Consequently, as illustrated in Fig. 14, the tip 11a of the syringe 11 is inserted into the communication hole 10d, and the syringe 11 is rotated, thus the thread shape 11b of the syringe 11 and the thread shape 10aa of the locking part 10a are screwed together and connected, whereby the syringe 11 can be locked (fixed) and connected to the access port member 7.

The access port member 7 according to the present embodiment includes: the body 8 having the opening 8c; the valve 9 made of an elastic member covering the opening 8c and attached to the attachment part 8d of the body 8 in a press-fitted state, the valve 9 including the line segment-shaped slit 9a that makes a transition from a closed state to an open state when pressed by the tip 11a of the syringe 11 (connecting member) capable of collecting blood or injecting a drug; the cap part 10 fixed to the body 8 with the valve 9 interposed therebetween and the slit 9a facing outward; and the rib 9b integrally formed over the periphery of the slit 9a in the valve 9. The valve 9 is matched in shape and attached to the attachment part 8d in the body 8, and has an asymmetric shape about the extension direction u of the flow path 8b and the orthogonal direction v to the flow path, thus the valve 9 can be easily attached to the attachment part 8d of the body 8 while extending the slit 9a in a desired direction.

In particular, the body 8 is connected to a predetermined portion of the blood circuit for causing a patient's blood to extracorporeally circulate, thus the following effects can be achieved. Specifically, occurrence of a stagnation point on the rear surface of the valve 9 may cause blood clotting, thus such an occurrence needs to be reduced as much as possible. However, when the valve 9 is located closer to the flow route to prevent stagnation, the valve 9 cannot be sufficiently pressed by the tip 11a of the syringe 11, which makes it difficult to open the slit 9a. Thus, providing the valve 9 with the rib 9b as in the present embodiment allows the valve 9 to be sufficiently pressed by the tip 11a of the syringe 11, whereby both easiness of opening the slit 9a and less occurrence of a stagnation point can be achieved.

The valve 9 according to the present embodiment has the contour of a rectangle having the long side A and the short side B in a plan view, and the slit 9a extends in the direction parallel to the long side A of the rectangle, thus a compressive force in the direction in which the slit 9a is closed can be made larger than a compressive force in the opening direction, and a closed state of the slit 9a can be achieved with a stronger compressive force. Since the slit 9a extends in the extension direction of the flow path 8b, when the slit 9a is in an open state, it is possible to avoid prevention of flow of blood by a drooping area in the periphery of the slit 9a.

However, the inner contour dimensions of the attachment part 8d of the body 8 and the outer contour dimensions (the long side A, the short side B) of the valve 9 can be set as follows. For example as illustrated in Fig. 7, let X be the longitudinal dimension (the dimension of the area corresponding to the long side A) and Y be the transverse dimension (the dimension of the area corresponding to the short side B) of the inner contour of the attachment part 8d, then a compressive force (closing force) can be applied to the slit 9a by setting Y < B, and in this case, it may be difficult to attach the valve 9 to the attachment part 8d.

Thus, for example, as illustrated in Figs. 7 and 8, let r1 be the curvature of an inner corner R1 of the attachment part 8d, and r2 be the curvature of an outer corner R2 (the corner fitted into the inner corner R1) of the valve 9, then the outer corner R2 of the valve 9 can be easily fitted into the inner corner R1 of the attachment part 8d by setting X = A, or setting the compression rates (X/A, Y/B) of the long side and the short side of the valve 9 to X/A > Y/B with R1 < R2. With the outer corner R2 fitted into the inner corner R1, the valve 9 can be smoothly and easily attached to the attachment part 8d by fitting in the short side B of the valve 9. The settings described above can reduce the assembly man-hour for attaching the valve 9 to the attachment part 8d, and just placing the valve 9 in conformity with the attachment part 8d allows the valve 9 to be accurately attached only by a pressing force caused by welding when the cap part 10 is ultrasonically welded.

In addition, the valve 9 according to the present embodiment has the rib 9b integrally formed over the periphery of the slit 9a, thus connection work of the syringe 11 (connecting member) can be facilitated while preventing a failure in closing of the slit 9a. In addition, the cap part 10 according to the present embodiment includes the locking part 10a with the thread shape 10aa to be screwed and locked into the thread shape 11b of the syringe 11 (connecting member), thus the following effects can be achieved. Specifically, since the dimensions of the locking part 10a are strictly defined by a standard, the slit 9a of the valve 9 may not be sufficiently pressed by the tip 11a of the syringe 11 (connecting member), thus the slit 9a may not be sufficiently opened. However, when the valve 9 is moved to the side where the syringe 11 is connected to prevent such a failure (for example, when the front surface of the valve 9 is superficialized to the side where the syringe 11 is connected), the valve 9 is made thicker, making it further difficult to open the slit 9a. When the valve 9 is made thinner and the valve 9 is superficialized, a liquid stagnation point occurs in greater number on the flow route side of the valve 9, and stagnation of blood and liquid drug is likely to occur, and in particular, when blood stagnates, blood clotting may occur. Thus, a structure needs to be created in which the slit 9a is reliably opened and stagnation is unlikely to occur. Thus, according to the present embodiment, providing the valve 9 with the rib 9b allows the tip 11a of the syringe 11 to sufficiently press the valve 9, whereby both easiness of opening the slit 9a and prevention of a stagnation point can be achieved.

The connecting member to be used is not limited to the syringe 11, and may be another connecting member (for example, a connecting member attached to the distal end of an infusion tube) which has a tip capable of pressing the rib 9b, and causes a transition of the slit 9a from a closed state to an open state by pressing to enable blood collection or drug injection. The depressions 8e may not be formed in the body 8.

As long as the valve 9 has an asymmetric shape about the extension direction u of the flow path 8b and the orthogonal direction v to the flow path, for example, as illustrated in Fig. 27, the valve 9 may be the one having an elliptic shape, in which the slit 9a extends in a predetermined (the direction parallel to the extension direction u of the flow path 8b in Fig. 27), or having a different shape, in which the slit 9a extends in a predetermined (the direction parallel to the extension direction u of the flow path 8b in Fig. 27) as illustrated in Fig. 28.

In addition, the present invention is not limited to the one connected to a flow route for blood in the blood circuit, and may be connected to various flow routes (such as a liquid drug infusion line or a liquid drug delivery line serving as a flow route for a liquid drug) extending from the blood circuit. More specifically, in addition to the blood circuit for causing a patient's blood to extracorporeally circulate, the present invention is applicable to a circuit connected to a liquid delivery circuit, used for collection of blood flowing in the liquid delivery circuit, sampling of a liquid drug, and infusion of a liquid drug. For application to a liquid delivery circuit, an access port member may be used with one end thereof connected to an infusion bag and the other end connected to a connector coupled to an infusion line such as an indwelling needle or a blood circuit to enable drug injection and sampling, or an access port member may be used with connected to a liquid drug delivery circuit extended from a blood purifier such as the dialyzer 3 or various filters such as a blood adsorber, and a plasma separator to enable drug injection and sampling.

For application to a liquid delivery circuit, an access port member 12 having the configuration illustrated in Figs. 19 and 20 may be used. The access port member 12 includes: a body 13 having an opening 13b; a valve 9 made of an elastic member covering the opening 13b and attached to an attachment part of the body 13, the valve 9 including a slit 9a that makes a transition from a closed state to an open state when pressed by the tip 11a of the syringe 11 (connecting member) capable of collecting blood or injecting a drug; a cap part 10 fixed to the body 13 with the valve 9 interposed therebetween and the slit 9a facing outward, the cap part 10 having a locking part 10a with a thread shape 10aa to be screwed and locked into a thread shape 11b formed in the syringe 11; and a rib 9b integrally formed over the periphery of the slit 9a in the valve 9. The distal tip of a liquid delivery circuit H is to be connected to a connecting part 13a formed in the body 13. Note that the access port member 12 may be used instead of the connecting part K illustrated in Fig. 1.

Meanwhile, when the valve 9 according to the embodiment is attached to the attachment part 8d of the body 8, the valve 9 is compressed in a direction in which the slit 9a is closed; however, as illustrated in Fig. 29, the valve may include the cap part 10 in which an inclined surface M extending parallel to the slit 9a is formed. In this case, the external dimensions of the valve 9 are set to be equal to the dimensions of the attachment part 8d, and after the valve 9 is attached to the attachment part 8d, the cap part 10 is fixed to the body 8, causing the inclined surface M to press against the valve 9, thus the valve 9 can be compressed in a direction in which the slit 9a is closed.

As illustrated in Figs. 21 to 24, the access port member 7 may include a cap part G. The access port member 7 includes the same body 8 as illustrated in Figs. 6 to 8, and the valve 9 in which the slit 9a is formed, and the connecting portions 8a are to be connected to a flow route in a blood circuit or the like. The cap part G in this case includes a locking part Ga fixed to the body 8 with the valve 9 interposed therebetween and the slit 9a facing outward in an insertion hole Gb, the locking part Ga with a thread shape Gaa to be screwed and locked into a thread shape formed in a connecting member such as the syringe 11. The same inclined surface M as previously described is formed on the rear surface of the cap part G, and after the valve 9 is attached to the attachment part 8d, the cap part G is fixed to the body 8, causing the inclined surface M to press against the valve 9, thus the valve 9 is compressed in a direction in which the slit 9a is closed. The inclined surface M is formed parallel to the long side A of the valve 9, and the slit 9a of the valve 9 is formed parallel to the long side A.

In addition, as illustrated in Figs. 23 and 24, in the cap part G, protrusions Gca, Gcb formed at positions corresponding to depressions 8e of the body 8, and projection parts Gd formed at positions corresponding to notches N of the body 8 are integrally formed. As illustrated in Figs. 21 and 22, the protrusions Gca, Gcb have a projecting shape which is to be fitted into the depressions 8e when the cap part G is welded and fixed to the body 8, and protrusions Gca at positions adjacent to the long sides A of the valve 9, and protrusions Gcb at positions adjacent to the short sides B of the valve 9 are set to have different dimensions. The depressions 8e each serves as downgage, and include: depressions 8e corresponding to protrusions Gca adjacent to the long sides A; and depressions 8e corresponding to protrusions Gcb adjacent to the short sides B.

Thus, when the cap part G is attached to the attachment part 8d of the body 8, positioning in the rotational direction can be made by fitting the protrusions Gca, Gcb into the depressions 8e. However, when the positioning in the rotational direction is not correct at the time of attachment of the cap part G, a protrusion Gca having a larger size cannot be fitted into a depression 8e having a smaller size, and comes into contact with its peripheral surface, thus energy more than necessary is consumed at the time of ultrasonic welding, which makes it possible to inform of an error. In particular, the cap part G includes the inclined surface M that causes the valve 9 to be compressed in a closing direction of the slit 9a, thus positioning can be accurately made so that the inclined surface M becomes parallel to the slit 9a. Since the depressions 8e are formed in the body 8, the protrusions Gca, Gcb of the cap part G can be fitted thereinto, and positioning can be accurately made so that the inclined surface M becomes parallel to the slit 9a.

When the cap part G is welded and fixed to the body 8, if the positioning of the cap part G in the rotational direction is correct, each projection part Gd is opposed to the wall surface of a corresponding notch N as illustrated in Figs. 21 and 22. Thus, at the time of attachment of the cap part G, whether the positioning in the rotational direction is correct can be visually checked. As illustrated, the projection parts Gd are formed as a pair of right and left, but one of the pair may be formed, or alternatively, no projection part Gd may be formed.

In the first and second embodiments, the access port member is applied to a blood circuit in hemodialysis treatment, but may also be applied to other flow routes (such as a blood circuit installed in an apparatus used for e.g., hemodiafiltration, hemofiltration, AFBF, continuous renal replacement therapy, hemoadsorption, selective cytapheresis, simple plasma exchange, double filtration plasmapheresis, or plasma adsorption) for enabling purification treatment of a patient's blood.

A first aspect of the present invention provides an access port member including: a body 8 having an opening 8c; a valve 9 made of an elastic member covering the opening 8c and attached to an attachment part 8d of the body 8, the valve 9 including a slit 9a that makes a transition from a closed state to an open state when pressed by the tip 11a of a syringe 11 (connecting member) capable of collecting blood or injecting a drug; a cap part 10 welded and fixed to the body 8 with the valve 9 interposed therebetween and the slit 9a facing outward, the cap part 10 having a locking part 10a with a thread shape 10aa to be screwed and locked into a thread shape 11b formed in the syringe 11 (connecting member); and the depressions 8e formed at positions between the welding part Y1 of the body 8 and the attachment part 8d, or between the welding part Y2 of the cap part 10 and the clamp region of the valve 9. Thus, it is possible to avoid reduction in the durability of the valve 9 caused by welding and fixing of the cap part 10 to the body 8.

A second aspect of the present invention provides the access port member according to the first aspect, in which the valve 9 is matched in shape and attached to the attachment part 8d in the body 8, and has an asymmetric shape about the extension direction u of the flow path 8b and the orthogonal direction v to the flow path 8b. Thus, the valve 9 can be easily attached to the attachment part 8d of the body 8 while extending the slit 9a in a desired direction.

A third aspect of the present invention provides the access port member according to the first aspect, in which the valve 9 is made of an elastic member having a rectangular plan view, the attachment part 8d of the valve 9 in the body 8 is formed in a rectangular shape copying the shape of the valve 9, and the depression 8e is formed at multiple locations corresponding to the sides of the valve 9 attached to the attachment part 8d. Thus, the molten resin flowing from the welding parts Y1, Y2 can be reliably passed into the depression 8e.

A fourth aspect of the present invention provides the access port member according to the first or third aspect, in which the depression 8e serves as downgage of the body 8 or the cap part 10. Thus, both the effect (prevention of shrinkage) of the downgage, and the effect (maintenance of the durability of the valve 9) of passing the molten resin flowing from the welding parts Y1, Y2 into the depression 8e can be achieved.

A fifth aspect of the present invention provides an access port member including: a body 8 having an opening 8c; a valve 9 made of an elastic member covering the opening 8c and attached to an attachment part 8d of the body 8 in a press-fitted state, the valve 9 including a line segment-shaped slit 9a that makes a transition from a closed state to an open state when pressed by a tip 11a of the syringe 11 (connecting member) capable of collecting blood or injecting a drug; and a cap part 10 fixed to the body 8 with the valve 9 interposed therebetween and the slit 9a facing outward, the cap part 10 having a locking part 10a with a thread shape 10aa to be screwed and locked into a thread shape 11b formed in the syringe 11 (connecting member). The valve 9 is matched in shape and attached to the attachment part 8d in the body 8, and has an asymmetric shape about the extension direction u of the flow path 8b and the orthogonal direction v to the flow path 8b. Thus, the valve 9 can be easily attached to the attachment part 8d of the body 8 while extending the slit 9a in a desired direction.

A sixth aspect of the present invention provides the access port member according to the fifth aspect, in which the valve 9 has the contour of a rectangle having the long side A and the short side B in a plan view, and the slit 9a extends in the direction parallel to the long side A of the rectangle. Thus, a compressive force in the direction in which the slit 9a is closed can be made larger than a compressive force in the opening direction, and a closed state of the slit 9a can be achieved with a stronger compressive force.

A seventh aspect of the present invention provides the access port member according to the fifth or sixth aspect, in which the slit 9a extends in the extension direction of the flow path 8b. Thus, when the slit 9a is in an open state, it is possible to avoid prevention of flow of blood by a drooping area in the periphery of the slit 9a.

An eighth aspect of the present invention provides the access port member according to one of the fifth to seventh aspects, in which the valve 9 has a rib 9b integrally formed over the periphery of the slit 9a. Thus, connection work of the syringe 11 (connecting member) can be facilitated while preventing a failure in closing of the slit 9a.

A ninth aspect of the present invention provides the access port member according to one of the fifth to eighth aspects, in which the cap part 10 includes an inclined surface M that extends parallel to the slit 9a. Thus, positioning can be accurately made so that the inclined surface M becomes parallel to the slit 9a.

A tenth aspect of the present invention provides a method of manufacturing the access port member according to one of the first to ninth aspects, in which the welding part Y1 of the body 8 and the welding part Y2 of the cap part 10 are welded by being matched and melted. Thus, the cap part 10 can be fixed to the body 8 with high accuracy.

An eleventh aspect of the present invention provides the method of manufacturing the access port member, according to the tenth aspect, in which welding of the body 8 and the cap part 10 is ultrasonic welding that performs welding by applying ultrasonic waves to the welding parts (Y1, Y2). Thus, the body 8 and the cap part 10 can be fixed with ease and high accuracy.

A twelfth aspect of the present invention provides a flow route to be connected to the body 8 according to one of the first to eleventh aspects, the flow route comprising a blood circuit configured to cause a patient's blood to extracorporeally circulate, a liquid delivery circuit configured to inject a liquid drug or blood to a patient, or a liquid drug delivery circuit configured to cause a liquid drug to flow. Consequently, the present invention is applicable to a blood circuit, a liquid delivery circuit configured to inject a liquid drug or blood to a patient, or a liquid drug delivery circuit configured to cause a liquid drug to flow.

### Industrial Applicability

The present invention is also applicable to any blood purification apparatus having a different appearance, additional functions, or the like within the spirit of the present invention.

### Reference Signs List

- 1: ARTERIAL BLOOD CIRCUIT
- 2: VENOUS BLOOD CIRCUIT
- 3: DIALYZER (BLOOD PURIFIER)
- 4: BLOOD PUMP
- 5: AIR-TRAP CHAMBER
- 6: DIALYSIS APPARATUS BODY
- 7: ACCESS PORT MEMBER
- 8: BODY
- 8a: CONNECTING PORTION
- 8b: FLOW PATH
- 8c: OPENING
- 8d: ATTACHMENT PART
- 8e: DEPRESSION
- 9: VALVE
- 9a: SLIT
- 9b: RIB
- 9ba: TIP SURFACE
- 9bb: LATERAL SURFACE
- 10: CAP PART
- 10a: LOCKING PART
- 10aa: THREAD SHAPE
- 10b: OUTER PERIPHERAL EDGE
- 10c: INNER PERIPHERAL DEPRESSION
- 10d: COMMUNICATION HOLE
- 11: SYRINGE (CONNECTING MEMBER)
- 11a: TIP
- 11b: THREAD SHAPE
- 12: ACCESS PORT MEMBER
- 13: BODY
- 13a: CONNECTING PART
- 13b: OPENING
- A: LONG SIDE
- B: SHORT SIDE
- S1: (PRE-WELDING) CLOSED SPACE
- S2: (POST-WELDING) CLOSED SPACE
- Y1: WELDING PART (OF BODY)
- Y2: WELDING PART (OF CAP PART)
- La: LIQUID DRUG INFUSION LINE
- H: LIQUID DELIVERY CIRCUIT

## Claims

1. An access port member comprising:
a body having an opening;
a valve made of an elastic member covering the opening and attached to an attachment part of the body, the valve including a slit that makes a transition from a closed state to an open state when pressed by a tip of a connecting member capable of collecting blood or injecting a drug;
a cap part welded and fixed to the body with the valve interposed therebetween and the slit facing outward, the cap part having a locking part with a thread shape to be screwed and locked into a thread shape formed in the connecting member; and
a depression formed at a position between a welding part of the body and the attachment part, or between a welding part of the cap part and a clamp region of the valve.

2. The access port member according to Claim 1,
wherein the valve is matched in shape and attached to the attachment part in the body, and has an asymmetric shape about an extension direction of a flow path and an orthogonal direction to the flow path.

3. The access port member according to Claim 1,
wherein the valve is made of an elastic member having a rectangular plan view, the attachment part of the valve in the body is formed in a rectangular shape copying a shape of the valve, and depressions are formed at multiple locations corresponding to sides of the valve attached to the attachment part.

4. The access port member according to Claim 1 or 3,
wherein the depression serves as downgage of the body or the cap part.

5. An access port member comprising:
a body having an opening;
a valve made of an elastic member covering the opening and attached to an attachment part of the body in a press-fitted state, the valve including a line segment-shaped slit that makes a transition from a closed state to an open state when pressed by a tip of a connecting member capable of collecting blood or injecting a drug; and
a cap part fixed to the body with the valve interposed therebetween and the slit facing outward, the cap part having a locking part with a thread shape to be screwed and locked into a thread shape formed in the connecting member,
wherein the valve is matched in shape and attached to the attachment part in the body, and has an asymmetric shape about an extension direction of a flow path and an orthogonal direction to the flow path.

6. The access port member according to Claim 5,
wherein the valve has a contour of a rectangle having a long side and a short side in a plan view, and the slit extends in a direction parallel to the long side of the rectangle.

7. The access port member according to Claim 5 or 6,
wherein the slit extends in the extension direction of the flow path.

8. The access port member according to any one of Claims 5 to 7,
wherein the valve has a rib integrally formed over a periphery of the slit.

9. The access port member according to any one of Claims 5 to 8,
wherein the cap part includes an inclined surface that extends parallel to the slit.

10. A method of manufacturing the access port member according to any one of Claims 1 to 9,
wherein the welding part of the body and the welding part of the cap part are welded by being matched and melted.

11. The method of manufacturing the access port member, according to Claim 10,
wherein welding of the body and the cap part is ultrasonic welding that performs welding by applying ultrasonic waves to the welding parts.

12. A flow route comprising: a blood circuit configured to cause a patient's blood to extracorporeally circulate; a liquid delivery circuit configured to inject a liquid drug or blood to a patient; or a liquid drug delivery circuit configured to cause a liquid drug to flow, the flow route being connected to the body according to any one of Claims 1 to 11.
